# EUROPEAN PATENT APPLICATION

(11) **EP 1 872 810 A1**
(43) Date of publication of application: **02.01.2008**
(21) Application number: 07110589.4
(22) Date of filing: 19.06.2007
(51) Int. Cl.: A61M 1/00

(54) **TOP AND BOTTOM CLAMPING FOR A SURGICAL CASSETTE**

(30) Priority: 29.06.2006 US 477214
(71) Applicant: ALCON INC., CH-6331 Hunenberg (CH)
(72) Inventor: Hopkins, Mark, Mission Viejo, CA 92691 (US); Williams, David, Newport Beach, CA 92663 (US); King, Nicolei, Aliso Viejo, CA 92656 (US)
(74) Representative: Hanna, Peter William Derek

(57) **Abstract**

Embodiments of the present invention provide top and bottom clamping of a surgical cassette (150). One embodiment of the present invention includes a surgical cassette adapted for use in a surgical system having a first side (170) (e.g., an inner side) and second side (180) (e.g., an outer side). The surgical cassette, according to one embodiment, comprises a body portion (155,205) configured to interface on its first side with a surgical console (100) during use, a first clamping portion (195) projecting from the top of the body portion configured to contact a first clamp rail (182) on the cassette's second side during use and a second clamping portion (190) projecting from the bottom of the body portion configured to contact a second clamp rail (142) on the cassette's second side during use.

## Description

### TECHNICAL FIELD OF THE INVENTION

The present invention relates to surgical systems. More particularly, the present invention relates to fluidics subsystems of a surgical system. Even more particularly, the present invention relates to a system for clamping surgical cassettes used in ophthalmic surgical systems.

### BACKGROUND OF THE INVENTION

The human eye can suffer a number of maladies causing mild deterioration to complete loss of vision. While contact lenses and eyeglasses can compensate for some ailments, ophthalmic surgery is required for others. Generally, ophthalmic surgery is classified into posterior segment procedures, such as vitreoretinal surgery, and anterior segment procedures, such as cataract surgery. More recently, combined anterior and posterior segment procedures have been developed.

The surgical instrumentation used for ophthalmic surgery can be specialized for anterior segment procedures or posterior segment procedures or support both. In any case, the surgical instrumentation often requires the use of associated consumables such as surgical cassettes, fluid bags, tubing, hand piece tips and other consumables.

A surgical cassette can provide a variety of functions depending on the procedure and surgical instrumentation. For example, surgical cassettes for cataract surgeries (e.g., phacoemulsification procedures) help manage irrigation and aspiration flows into and out of a surgical site. Surgical cassettes can also provide support for fluid bags, a manifold for directing vacuum/pressure to surgical instrumentation, and other functionality.

Cassettes are generally coupled to the surgical instrumentation at a cassette receiving site by vertical clamping rails. When the cassette is inserted into the cassette receiver, a clamp closes on the cassette to hold the cassette in place. During operation, the surgical cassette can experience a significant amount of force in the clamping area. This force can be the result of the clamps counteracting a force applied by a peristaltic pump pushing near the center of the cassette or other forces.

A clamping mechanism that clamps a cassette on the sides suffers several shortcomings. For example, the width of the cassette must be increased to provide an area of the clamp to engage the cassette. Moreover, during operation, side clamps can interfere with sensors located along the sides of a cassette.

Therefore, a need exists for a surgical cassette and system for clamping a surgical cassette that can reduce or eliminate the problems of prior art side-clamping cassettes and clamping systems.

### SUMMARY OF THE INVENTION

A clampable surgical cassette and a surgical system including a cassette clamping mechanism are provided by the invention, in accordance with claims which follow. One embodiment of the present invention includes a surgical cassette adapted for use in a surgical system having a first side (e.g., an inner side) and second side (e.g., an outer side). The surgical cassette, according to one embodiment, comprises a body portion configured to interface on its first side with a surgical console during use, a first clamping portion projecting from the top of the body portion configured to contact a first clamp rail on the cassette's second side during use and a second clamping portion projecting from the bottom of the body portion configured to contact a second clamp rail on the cassette's second side during use.

Another embodiment of the present invention includes a cassette having a body portion to house at least a portion of a fluidics management system for an ophthalmic surgery process, a first clamping portion attached to the top of the body portion and a second clamping portion attached to the bottom of the body portion. The surgical cassette is adapted for insertion into a cassette receiver in a surgical console.

Yet another embodiment of the present invention includes a surgical system comprising a surgical console for an ophthalmic surgical procedure and a surgical cassette. The surgical console comprises a cassette receiver to receive surgical cassettes and a clamping mechanism. The clamping mechanism further comprises a top rail rotatable about a first horizontal axis and a bottom rail rotatable about a second horizontal axis.

The surgical cassette has a first side and second side opposite the first side. The surgical cassette, according to one embodiment, comprises a body portion housing at least a portion of a fluidics management system for the ophthalmic surgical procedure, a top clamping portion to contact the top clamp rail on the second side of the surgical cassette and a bottom clamping portion attached to the body housing to contact the bottom clamp rail on the second side of the surgical cassette. The body portion is configured to interface with the surgical console on the first side during use.

According to one embodiment, the top and bottom rails can include inner and outer fingers to contact the cassette. For example, the outer fingers can contact the surgical cassette on the top and bottom clamping portions to hold the surgical cassette in place. The inner fingers can contact the clamping portions of the surgical cassette to push the surgical cassette out of the console during a cassette release process.

Embodiments of the present invention provide an advantage by reducing the width of a surgical cassette and hence the width of the console designed to accommodate the cassette.

Embodiments of the present invention provide another advantage by clamping a cassette near the area of the highest applied load.

Embodiments of the present invention provide yet another advantage by avoiding clamping in areas used for sensors which sense the levels of liquids that are contained in the surgical cassette.

### BRIEF DESCRIPTION OF THE FIGURES

A more complete understanding of the present invention and the advantages thereof may be acquired by referring to the following description, taken in conjunction with the accompanying drawings in which like reference numbers indicate like features and wherein:
FIGURE 1 is a diagrammatic representation of one embodiment of a surgical console;
FIGURE 2 is a diagrammatic representation of one embodiment of a cassette receiver;
FIGURE 3 is a diagrammatic representation of one embodiment of a surgical cassette;
FIGURE 4 is a diagrammatic representation of one embodiment of a surgical cassette in a cassette receiver;
FIGURE 5 is a diagrammatic representation illustrating a cross-sectional view of one embodiment of a cassette in a cassette receiver;
FIGURE 6 is a detailed view of one embodiment of a cassette engaged with a clamp;
FIGURE 7 is a diagrammatic representation of one embodiment of a cassette section including a clamping portion;
FIGURE 8 is a diagrammatic representation of a profile of one embodiment of a surgical cassette; and
FIGURES 9 and 10 are diagrammatic representations of one embodiment of a clamping mechanism.

### DETAILED DESCRIPTION

Preferred embodiments of the invention are illustrated in the FIGURES, like numerals being used to refer to like and corresponding parts of the various drawings.

Embodiments of the present invention provide a surgical system and surgical cassette in which the surgical cassette is clamped at the top and bottom of the cassette. According to one embodiment of the present invention, a surgical console includes a clamp having clamp rails that each rotate about a horizontal axis to clamp a surgical cassette along the cassette's top and bottom edges. Clamp fingers can extend from each clamp rail. Inner clamp fingers can help locate the cassette during insertion of the cassette and release the cassette during a release process. Outer clamp fingers contact and clamp the cassette. When the clamp rails rotate the outer fingers contact the outer facing side of the cassette to push the cassette towards the console. The cassette can include a clamping portion, such as lip or step to receive the clamp fingers.

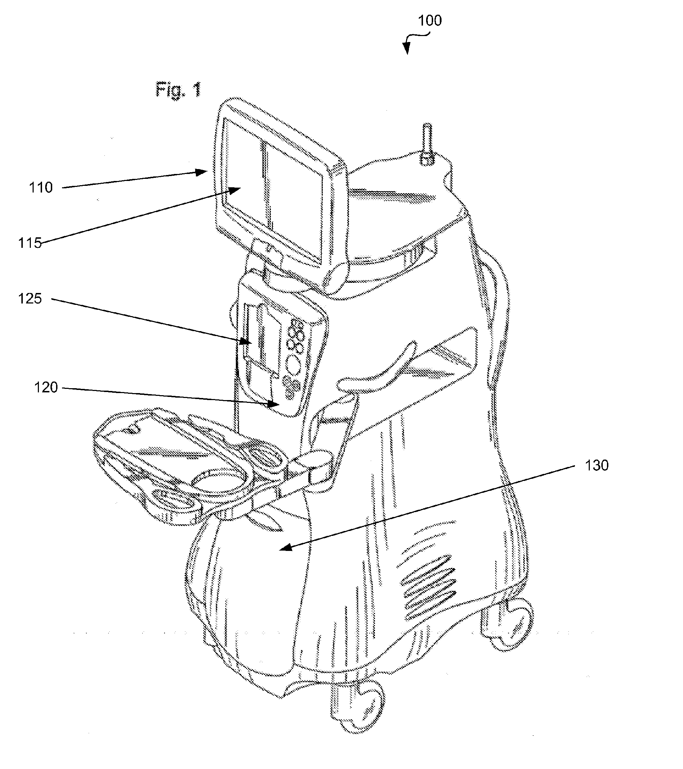

FIGURE 1 is a diagrammatic representation of one embodiment of an ophthalmic surgical console 100. Surgical console 100 can include a swivel monitor 110 that has touch screen 115. Swivel monitor 110 can be positioned in a variety of orientations for whomever needs to see touch screen 115. Swivel monitor 110 can swing from side to side, as well as rotate and tilt. Touch screen 115 provides a user interface, such as a graphical user interface ("GUI"), that allows a user to interact with console 100.

Surgical console 100 also includes a connection panel 120 used to connect various tools and consumables to surgical console 100. Connection panel 120 can include, for example, a coagulation connector, balanced salt solution receiver, connectors for various hand pieces and a fluid management system ("FMS") or cassette receiver 125. Surgical console 100 can also include a variety of user friendly features, such as a foot pedal control (e.g., stored behind panel 130) and other features.

In operation, a cassette (not shown) can be placed in cassette receiver 125. A clamp in surgical console 100 clamps the cassette in place to minimize movement of the cassette during use. The clamp can clamp the top and bottom of the cassette, the sides of the cassette or otherwise clamp the cassette.

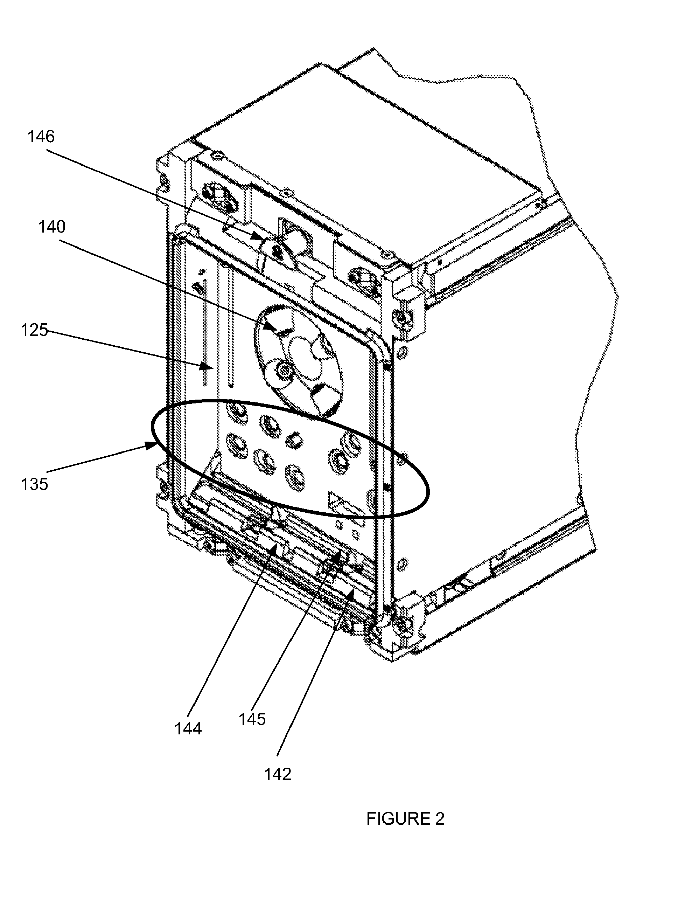

FIGURE 2 is a diagrammatic representation of one embodiment of cassette receiver 125 without a cassette. Cassette receiver 125 can have various input and output ports (indicated generally at 135) to receive fluids (i.e., liquids and gasses) from the surgical cassette. Cassette receiver 125 can further include an opening to allow peristaltic pump rollers 140 to contact the surgical cassette during operation. One embodiment of a peristaltic pump and complimentary cassette is described in United States Patent Application No. 6,293,926 to Sorensen.

The surgical cassette, in the embodiment of FIGURE 2, is held in place by a clamp having a bottom rail 142 and a top rail (not shown). Each rail can have outer clamping fingers (e.g., clamp finger 144) that contact the cassette in corresponding clamping zones and inner clamping fingers (e.g., clamp finger 145) to locate the cassette during insertion and push the cassette out of the cassette receiver during release. A release button 146 can be pressed to initiate release of the cassette from the clamp. Depending on the surgical console 100, the cassette release process can include several steps, including venting of pressure or fluids, disengaging the clamps or other steps. The configuration shown in FIGURE 2 is provided by way of example. The form factor of cassette receiver 125, placement and number of input/output ports and other features of cassette receiver 125 can depend on the surgical console 100, surgical procedure being performed or other factors.

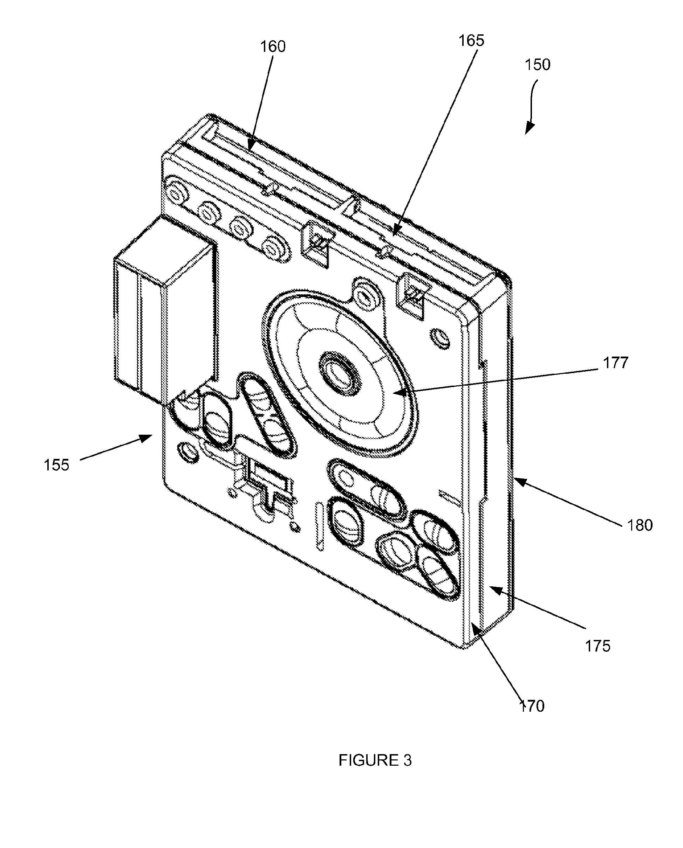

FIGURE 3 is a diagrammatic representation of one embodiment of a surgical cassette 150. Cassette 150 can provide a closed system fluidic device that can be discarded following a surgical procedure. Cassette 150 can include a cassette body 155 and portions that interface with the clamp (e.g., indicated generally at clamping zones 160 and 165) projecting from the cassette body 155. In the embodiment shown, cassette 150 is formed from three primary sections: an inner or surgical console interface section 170 that faces the surgical console when cassette 150 is inserted into surgical console 100, a middle section 175 and a back plate 180. The various sections of cassette 150 can be coupled together via a press fit, interlocking tabs, chemical bonding, thermal bonding, mechanical fasteners or other attachment mechanism known in the art. In other embodiments, cassette 150 can be formed of a single piece or multiple pieces.

Surgical console interface section 170 can face the console during use and provide an interface for fluid flow channels (e.g., flow channel 177 for the peristaltic pump provided by an elastomeric pump membrane), valves (e.g., irrigation/aspiration valves), pressure sensors and other features to manage fluid flow. Cassette 150 can also attach to a fluid bag (not shown) to collect fluids during a procedure.

In operation, cassette 150 is held in place in cassette receiver 125 by clamp rails that contact cassette 150 on the top and bottom of cassette 150. For example, the upper clamp rail will contact cassette 150 in clamping zone 160 and clamping zone 165 while the bottom clamp rail (e.g., bottom clamp rail 142) will contact cassette 150 at similar bottom clamping zones.

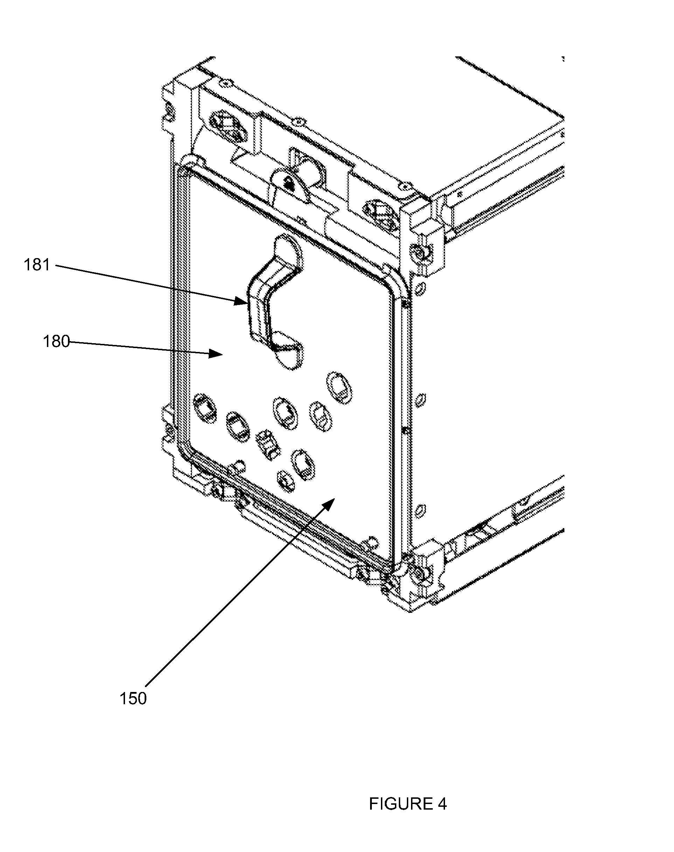

FIGURE 4 is a diagrammatic representation of cassette 150 inserted in cassette receiver 125. As can be noted from FIGURE 4, front cover 180 can include a handle 181 for one-handed insertion and removal of cassette 150.

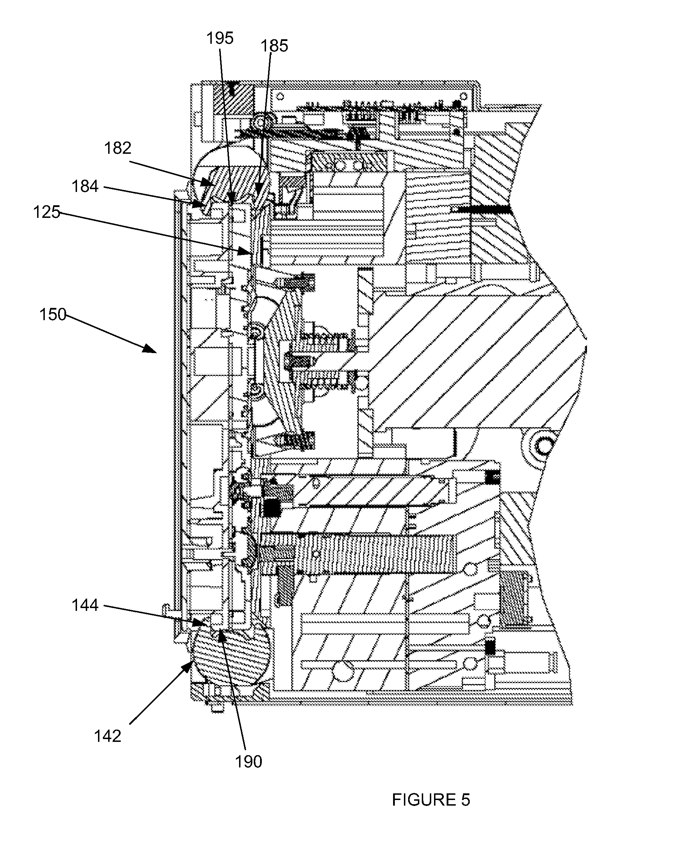

FIGURE 5 is a cross-section of one embodiment of cassette 150 inserted into cassette receiver 125. Cassette 150 is held in place by a clamp on the top and bottom. In the embodiment of FIGURE 5, the clamp includes lower clamp rail 142 and upper clamp rail 182, though in other embodiments the clamp can contact cassette 150 in other areas. When cassette 150 is initially inserted, cassette 150 pushes on the inner clamp fingers (e.g., clamp finger 145 and clamp finger 185) so that clamp rails rotate and the outer clamping fingers (e.g., clamp finger 144 and clamp finger 184) contact cassette 150 in the clamping zones. For example, clamp finger 144 contacts cassette 150 at clamping portion 190 while clamp finger 184 can contact cassette 150 and clamping portion 195. As cassette 150 is inserted into receiving portion 125, additional rotation can be imparted to the clamp rails directly or indirectly by a motor, air cylinder, linear actuator, solenoid or by a combination of any of these or other linear or rotary driver. To release the cassette, the clamp rails rotate in the opposite direction and the inner clamp fingers push the cassette away from the console. When inserted, surgical console interface section 170 can contact surgical console 100 such that, for example, peristaltic pump rollers 140 can squeeze flow channel 177.

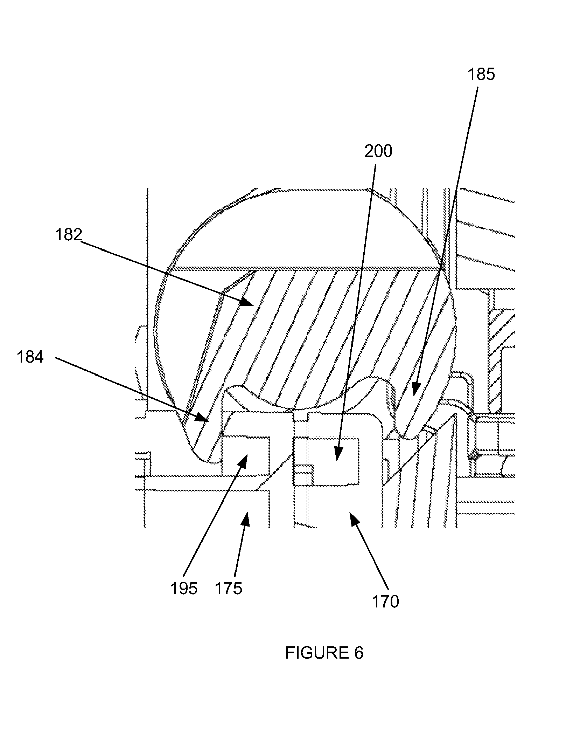

In the embodiment of FIGURE 5, the clamp fingers push cassette 150 towards surgical console 100 to hold cassette 150 in place. FIGURE 6 is a detail view of cassette 150 clamped by clamp rail 182. In the embodiment of FIGURE 6, middle section 175 defines a portion of cassette body 155 and console interface section 170 defines a portion of cassette body 155. Projecting from cassette body 155 (shown in FIGURE 3) is clamping portion 195 to engage with clamp finger 184 of clamp rail 182. Clamping portion 195 can be any suitable structure to engage clamp finger 184, such as a lip. It can be noted from FIGURE 6, that each of middle section 175 and console interface section 170 can include sections of clamping portion 195 (e.g., middle section 175 includes an outer section of clamping portion 195 and console interface section 170 includes an inner section of clamping portion 195).

During use, outer rail finger 184 can push on the outer side of clamping portion 195 to hold cassette 150 in place. During the release process, rail finger 185 can push on the inner side of clamping portion 195 to push cassette 150 out of the cassette receiver.

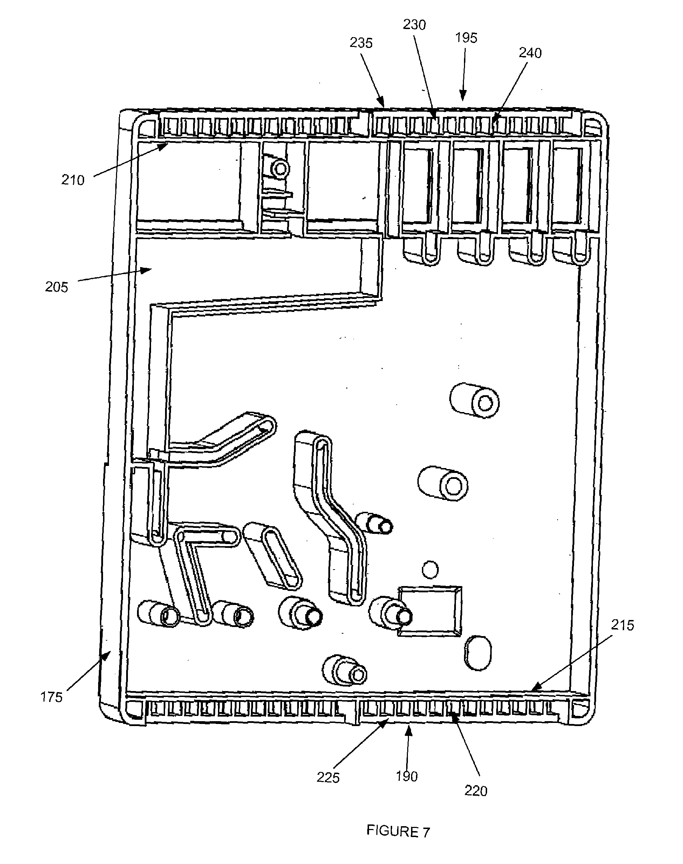

FIGURE 7 is a diagrammatic representation of one embodiment of middle section 175 of cassette 150. Middle section 175 can include a body portion 205 to define a portion of cassette body 155 (shown in FIGURE 3). Body portion 205 can include outer walls 210 and 215. In this example, body outer wall 210 is a top wall and body outer wall 215 is a bottom wall. Clamping portion 190 projects from body portion 205 and includes a set of ribs 220 transversely disposed between outer wall 215 and end wall 225. Similarly, clamping portion 195 projects from body portion 205 and includes a set of ribs 230 transversely disposed between outer wall 210 and end wall 235. The end faces of the ribs (e.g., end face 240) can contact the clamp (e.g., the clamping fingers) during use. According to other embodiments, the ribs can be behind a clamp interfacing wall configured to contact the clamp during use.

While FIGURE 7 illustrates the ribs as evenly spaced ribs with generally rectangular cross sections, the ribs can be otherwise disposed and shaped. In general, the ribs can be formed of a material and shaped such that the ribs are stable when loaded. Each rib can be made of a plastic such that the rib will deform predictably in the elastic region and will flow plastically when the material yield point is reached. As a rib deforms in the elastic region, other ribs can engage the clamp (or deform further if already contacting the clamp) to distribute the load. Similarly, if a rib deforms plastically, adjacent ribs can engage the clamp (or deform further) to distribute the load.

Put another way, because the clamp and cassette may not be parallel due to machining and assembly tolerances, the load profile imparted by the clamp may be non-uniform. The clamping portion of cassette 150 can conform to the load profile (e.g., by the ribs in the areas of higher loads deforming more) to distribute the load. Consequently, a non-uniformly distributed high load can be distributed in the clamping portion without causing catastrophic failure to cassette 150. The ribs can be sized and shaped depending on the expected load the cassette will experience.

While FIGURE 7 illustrates one embodiment of middle section 175, similar sections of a clamping portion can be included in console interface section 170 or otherwise included in cassette 150. Body portion 205, clamping portion 190 and clamping portion 195 can be a unitary piece of injection molded plastic, separate assemblies coupled together or otherwise attached. Additionally, FIGURE 7 is provided by way of example and the cassette clamping portions can have any suitable structure for contacting the clamp rails.

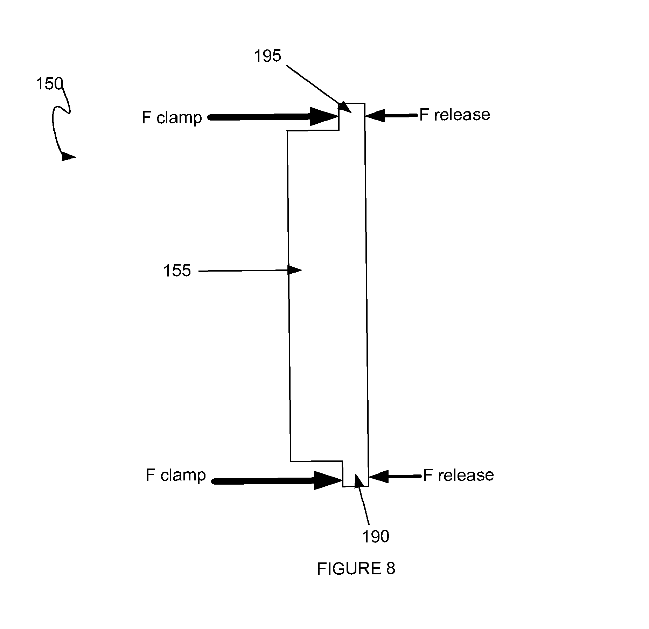

FIGURE 8 is a diagrammatic representation of a profile of a cassette 150 suitable for top and bottom clamping. In the embodiment of FIGURE 8, cassette 150 includes cassette body portion 155 to house one or more components of a fluidics management system for an ophthalmic surgical procedure. A top clamping portion 195 and a bottom clamping portion 190 are attached to body portion 155 (e.g., through boding, as a unitary piece of plastic or otherwise attached). The clamping portions, in this embodiment, are lips or steps that receive the clamp rails. During use, the clamp rails push on outer side, relative to the surgical console, of the clamping portions (represented by the large force arrows) to hold cassette 150 in place. As the cassette is released, the clamp rails can push on the inner side the clamping portions (represented by the smaller force arrows). Thus, the force to hold cassette 150 in place and release cassette 150 is applied at the top and bottom of cassette 150. The clamping portions can be compliant clamping portions utilizing a force distributing structure as described above or can be otherwise configured to contact the clamp rails during use.

Clamping cassette 150 on the top and bottom allows the width of cassette 150 to be reduced. This can provide an advantage for systems that are space constrained in the width dimension. Additionally, some surgical cassettes require the use of level sensors in the surgical console which are critical to the function of some fluidics systems. These sensors are typically located in the surgical console along the sides of the cassette where the appropriate fluid chambers are typically located. Clamping cassette 150 on the top and bottom frees the sides of cassette 150 for use of level sensors or other components of the fluidics system and eliminates or reduces stress on such components located near the sides of cassette 150. Additionally, clamping cassette 150 on the top and bottom provides clamping in or near the areas of the highest applied load on cassette 150 during use.

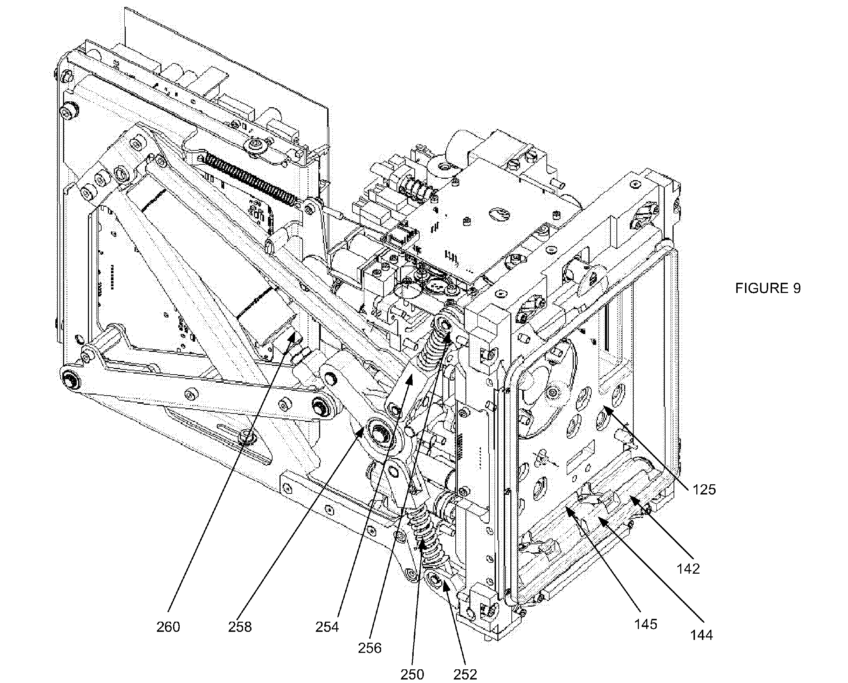

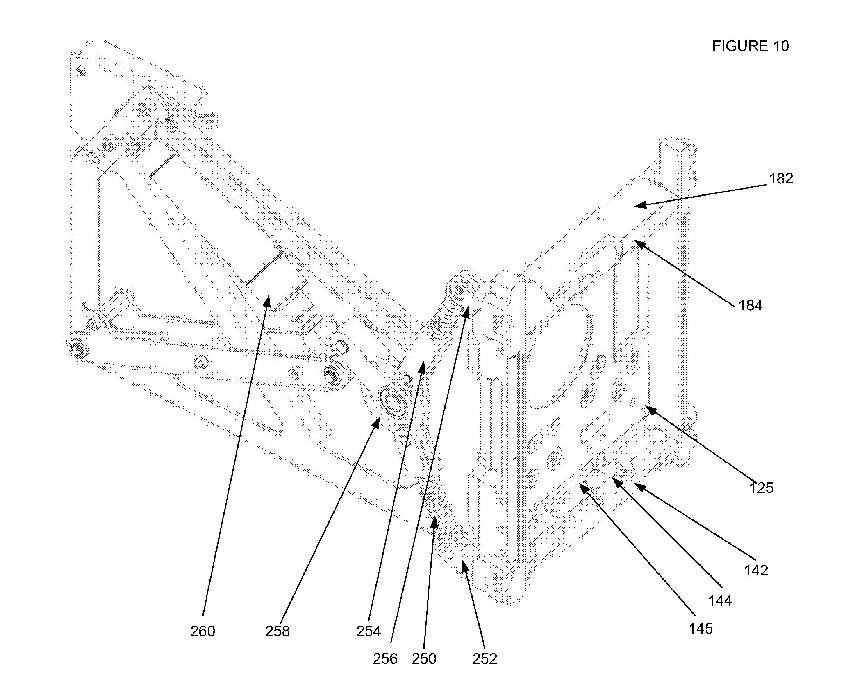

FIGURES 9 and 10 are diagrammatic representations of one embodiment of a system for clamping a surgical cassette on the top or bottom of the surgical cassette. As shown in FIGURE 9, a surgical console can include cassette receiver 125 and a clamping mechanism to hold the surgical cassette in place. The clamping mechanism can include a bottom clamp rail 142 and a top clamp rail 182 (shown in FIGURE 10). Each clamp rail can include inner and outer clamping fingers. For example, clamp rail 142 can include outer finger 144 and inner finger 145. The clamping mechanism can further include a clamp arm 250 coupled to pivot arm 252 of clamp rail 142 and clamp arm 254 coupled to pivot arm 256 of clamp rail 182. Clamp arms 250 and 254 can be compression links that include springs and reciprocating sockets to prevent overtorquing of clamp rails 142 and 182.

The opposite ends of clamping arms 252 and 254 are coupled to lobes of an actuator wheel 258. According to one embodiment, clamping arms 252 and 254 are eccentrically connected to actuator wheel 258. A third lobe of actuator wheel 258 is coupled to air cylinder 260 that expands and contracts. Air cylinder 260 can be eccentrically coupled to actuator wheel 258 directly or through a linkage.

In operation, as air cylinder 260 extends, actuator wheel 258 will move forward and rotate clockwise. The motion of actuator wheel 258 will cause movement of clamping arm 252 and clamping arm 254, thereby causing rotation of clamp rails 142 and 182. As actuator wheel 258 rotates clockwise from the perspective of FIGURES 9 and 10 and moves forward, upper rail 182 will rotate clockwise and lower rail 142 will rotate counter clockwise. If a surgical cassette is in place, this will cause the upper and lower outer clamp fingers to press the cassette into receiver 125. If, on the other hand, air cylinder 160 contracts, actuator wheel 258 will move back and rotate counterclockwise from the perspective of FIGURES 9, and 10, causing clamp rail 182 to rotate counterclockwise and clamp rail 142 to rotate clockwise. In this case, if a cassette is in place, inner clamping fingers (e.g., finger 145) will push the cassette out of cassette receiver 125.

While the example of air cylinder 160 is used to provide a force to ultimately rotate clamp rails 142 and 182, motion can be provided by a motor, linear actuator, solenoid, or other suitable mechanism. While a particular clamping mechanism is depicted in FIGURES 9 and 10, other embodiments of the present invention can utilize other clamping mechanism to clamp a surgical cassette on the top and bottom.

Thus, the clamping mechanism includes clamp rails that each rotate about a horizontal axis. The clamp rails can be located on the top and bottom of a cassette receiving area to clamp the cassette along its top and bottom edges (e.g., along corresponding clamping portions). The clamp rails can have extended fingers along their inner and outer edges, which contact the cassette. The fingers along the inner edge can be used to locate the cassette during insertion and eject the cassette during the cassette release process. The fingers along the outer edge can be used to contact and clamp the cassette. When the clamp rails are rotated to clamp the cassette, the outer clamp fingers approach and contact the cassette. The cassette has clamp receiving portions (e.g., steps) along its top and bottom edges to accept the outer fingers of the clamp rails for proper contact during clamping.

The location of the clamp rails at the top and bottom of the cassette allows the width of the cassette to be reduced. Moreover as the top and bottom of the cassette are typically subjected to the highest local loads during use, embodiments of the present invention allow clamping to occur near the areas of highest applied loading. Additionally, clamping the cassette on the top and bottom leaves the side of the cassette free for various components of the fluidics management system. Particularly, level sensors can be located near the sides of the cassette where the appropriate fluid chambers are typically located.

While the present invention has been described with reference to particular embodiments, it should be understood that the embodiments are illustrative and that the scope of the invention is not limited to these embodiments. Many variations, modifications, additions and improvements to the embodiments described above are possible. It is contemplated that these variations, modifications, additions and improvements fall within the scope of the invention as detailed in the following claims.

## Claims

1. A surgical cassette (150) having a first side (170) and second side (180) adapted for use in a surgical system (100) comprising:
a body portion (155,205) configured to interface on the first side (170) with a surgical console during use;
a first clamping portion (195) projecting from the top of the body portion configured to contact a first clamp rail (182) on the second side during use; and
a second clamping portion (190) projecting from the bottom of the body portion configured to contact a second clamp rail (142) on the second side during use.

2. The surgical cassette of the Claim 1, wherein the first clamping portion (195) comprises a first lip.

3. The surgical cassette of Claim 2, wherein the second clamping portion (190) comprises a second lip.

4. The surgical cassette of Claim 3, wherein the first clamping portion (195), second clamping portion (190) and the body portion (155) are formed from a unitary piece of plastic.

5. The surgical cassette of Claim 1, wherein the first clamping portion (195) and second clamping portion (190) comprise at least two sections.

6. The surgical cassette of any one of claims 1 to 5, wherein the body portion (155) is adapted to house at least a portion of a fluidics management system (140, 177) for an ophthalmic surgery process and wherein the surgical cassette (150) is adapted for insertion into a cassette receiver (125) in a surgical console (100).

7. A surgical system comprising:
a surgical console (100) for a ophthalmic surgical procedure, the surgical console comprising:
a surgical cassette receiver (125) to receive surgical cassettes (150);
a clamping mechanism, the clamping mechanism further comprising:
a top rail (182) rotatable about a first horizontal access;
a bottom rail (142) rotatable about a second horizontal access; a surgical cassette (150) having a first side (170) and second side (180) opposite the first side, the surgical cassette comprising:
a body portion (155,205) housing at least a portion of fluidics management system (140, 177) for the ophthalmic surgical procedure, wherein the body portion is configured to interface with the surgical console on the first side (170) during use;
a top clamping portion (195) attached to the body portion to contact the top clamp rail (182) on the second side of the surgical cassette; and
a bottom clamping portion (190) attached to the body portion to contact the bottom clamp rail (142) on the second side of the surgical cassette.

8. The surgical system of Claim 7, wherein the top clamping portion (195) of the surgical cassette (150) comprises a first lip.

9. The surgical system of Claim 8, wherein the bottom clamping portion (190) of the surgical cassette (150) comprises a second lip.

10. The surgical system of Claim 7, wherein:
the top rail (182) further comprises at least one top rail outer finger (184); and
the bottom rail (142) further comprises at least one bottom rail outer finger (144).

11. The surgical system of Claim 10, wherein the clamping mechanism is configured to contact the top clamping portion (195) of the surgical cassette (150) with the at least one top rail outer finger (184) and contact the bottom clamping portion (190) of the surgical cassette with the at least one bottom rail outer finger (144) when the surgical cassette is inserted into the surgical cassette receiver (125).

12. The surgical system of Claim 7, wherein the at least one top rail outer finger (184) contacts the top clamping portion (195) of the surgical cassette on the second side of the surgical cassette (150) and the at least one bottom rail outer finger (144) contacts the bottom clamping portion (190) of the surgical cassette on the second side of the surgical cassette.

13. The surgical system of Claim 10, wherein
the top rail (182) further comprises at least one top rail inner finger (185); and
the bottom rail (142) further comprises at least one bottom rail inner finger (145).

14. The surgical system of Claim 13, wherein the at least one top rail inner finger (185) and the at least one bottom rail inner finger (145) are configured to locate the surgical cassette (150) during insertion.

15. The surgical system of Claim 13, wherein the clamping mechanism is configured to push the surgical cassette (150) with the at least one top rail inner finger (185) and the at least one bottom rail inner finger (145) during a release process.

16. The surgical system of Claim 13, wherein the at least one top rail inner finger (185) contacts the top clamping portion (195) of the surgical cassette (150) on the first side of the surgical cassette and the at least one bottom rail inner finger (145) contacts the bottom clamping portion (190) of the surgical cassette on the first side of the surgical cassette.
